# EUROPÄISCHE PATENTSCHRIFT

(11) **EP 1 123 082 B1**
(45) Veröffentlichungstag und Bekanntmachung des Hinweises auf die Patenterteilung: **01.03.2006**
(21) Anmeldenummer: 99953787.1
(22) Anmeldetag: 12.10.1999
(51) Int. Cl.: A61K 8/49, A61K 9/06, A61K 9/70, A61K 31/421, A61Q 5/00, A61Q 7/02, A61K 31/4166, A61K 45/06, A61Q 7/00, A61Q 19/00

(54) **ZUBEREITUNGEN ZUR TOPISCHEN APPLIKATION VON ANTIANDROGEN WIRKSAMEN SUBSTANZEN**
PREPARATIONS FOR TOPICAL APPLICATION OF SUBSTANCES HAVING ANTIANDROGENIC EFFECT
PREPARATIONS POUR APPLICATION TOPIQUE DE SUBSTANCES A EFFET ANTIANDROGENE

(30) Priorität: 23.10.1998 DE 19848856; 12.01.1999 DE 19900749
(43) Veröffentlichungstag der Anmeldung: 16.08.2001
(73) Patentinhaber: Aventis Pharma S.A., 92160 Antony (FR)
(72) Erfinder: KRAEMER, Karl, Theodor, D-63225 Langen (DE); BOHN, Manfred, D-65719 Hofheim (DE)
(74) Vertreter: Rousseau, Pierrick Edouard
(86) Internationale Anmeldenummer: PCT/EP1999/007660
(87) Internationale Veröffentlichungsnummer: WO 2000/024366

(56) Entgegenhaltungen:
- EP-A- 0 540 854
- EP-A- 0 580 459
- EP-A- 0 743 311
- WO-A-97/23464
- FR-A- 2 751 965
- FR-A- 2 770 842

## Beschreibung

Die androgenetische Alopezie ist die häufigste Form des Haarverlustes, der sowohl bei Männern als auch bei Frauen auftreten kann. Unter dem Begriff "androgenetische Alopezie" werden Haarmangelzustände verstanden, deren Ursache eine genetisch determinierte Überempfindlichkeit der Haarwurzel auf 5α-Dihydrotestosteron ist.

Ein typisches Beispiel einer androgenetischen Alopezie ist die gewöhnliche Glatze des Mannes. Eine androgenetische Alopezie kann jedoch auch bei Frauen im geschlechtsreifen Alter - mit oder ohne die klinischen Merkmale der männlichen Glatze - auftreten.

Die Behandlung des androgenetischen Haarausfalls setzt die frühzeitige Unterbrechung der pathogenetischen Vorgänge voraus, die zur Rückbildung des Haarfollikels führen. Um eine Normalisierung des Haarzyklus, d.h. eine Verlängerung der Wachstumsphase der Haare zu erreichen, ist eine Verminderung der biologisch aktiven Androgenmenge am Follikel erforderlich. Wenn Endokrinopathien ausgeschlossen und Medikamente, die Testosteron oder andere androgen wirksame Substanzen enthalten, abgesetzt sind, ist die Hemmung der Androgenstimulation am Zielorgan notwendig. Zur Erreichung dieser Zielsetzung sind theoretisch zwei Wege denkbar: Erstens, die Aktivitätshemmung der 5α-Reduktase und damit Minderung der Umwandlung von Testosteron in 5α-Dihydrotestosteron, z.B. durch Östrogen, und zweitens eine Blockierung des Dihydrotestosteron-empfindlichen Rezeptorproteins, z.B. durch Antiandrogene.

Da alle systemischen Therapiemaßnahmen bei der androgenetischen Alopezie sich gegen die Androgenwirkung richten, ist ihre Anwendung bei gebährfähigen Frauen nur bei gleichzeitiger Kontrazeption möglich. Nach der Einführung der oralen Antikonzeptiva hat es sich gezeigt, daß je nachdem, ob man ein östrogenbetontes oder ein Präparat mit androgener Restwirkung verabreicht, der Verlauf einer androgenetischen Alopezie und ihrer Begleitsymtome günstig oder ungünstig beeinflußt wird.

In Ermangelung einer anderen, stärker wirksamen, gefahrlosen Alternative werden zur Behandlung der androgenetischen Alopezie bei Männern bislang östrogenhaltige Haarwässer verordnet. Bei Frauen wird diese Lokaltherapie als unterstützende Maßnahme empfohlen und das Hauptgewicht auf die systemische Behandlung gelegt.

Alle Patienten werden angewiesen, den noch behaarten Bereich der Kopfhaut zu behandeln und nicht die bereits kahlen Bezirke. In vielen Fällen gelingt es mit Hilfe dieser Lokalmaßnahmen die Schübe des Haarausfalls zu mildern oder zum Stillstand zu bringen.

Topisch wirksame Antiandrogene sind aus der französischen Patentschrift 2 693 461 und aus US 5,411,981 (4-[3-(4-Hydroxybutyl)-4,4-dimethyl-2,5-dioxo-1-imidazolidinyl]2-(tifluormethyl)-benzonitrile) sowie aus der PCT Anmeldung WO 98/05654 (3-Aryl-2,4-dioxo-oxazolidine) bekannt, stehen aber derzeitig noch nicht zu Therapiezwecken zur allgemeinen Verfügung.

Beide Substanzklassen zeigen nach topischer Applikation eine hohe Bindungsaffinität zum Androgenrezeptor an der Haarwurzel bei nahezu fehlender systemischer Aktivität.

Aufgrund der Substanz-inhärenten Teratogenität von Antiandrogenen mit Einfluß auf die Geschlechtsdifferenzierung im Spätstadium der Schwangerschaft sind die genannten Substanzen in Form von herkömmlichen wäßrig / alkoholischen Haarwässern wegen des Auftretens von Substanzausfällungen an der Applikationsstelle nach Verdunsten des Lösungsmittels und dem damit verbundenen toxikologischen Risikos der Substanzübertragung auf Schwangere nicht verwendbar. Ferner ist durch herkömmliche Zubereitungen zum Auftragen auf die Kopfhaut die verzögerte Wirkstofffreisetzung über einen längeren Zeitraum zur Vermeidung von hohen systemischen Wirkstoffkonzentrationen und dem damit einhergehenden Auftreten von systemischen antiandrogen Effekten nicht gewährleistet.

Um die in oben genannten Patenten antiandrogen Wirkstoffe für eine sichere und wirksame Therapie zur Verfügung stellen zu können, war es daher erforderlich, Formulierungen zu finden, die die beschriebenen Nachteile von herkömmlichen Kopfhautbehandlungsmitteln nicht aufweisen.

Die Aufgabe wird gelöst durch die erfindungsgemäßen Zubereitungen enthaltend ein oder mehrere topische Antiandrogene gemäß US 5,411,981 oder WO 98/05654, ein physiologisch verträgliches flüchtiges Lösemittel oder Lösemittelgemisch, einen Weichmacher und ein oder mehrere physiologisch unbedenkliche Filmbildner, die nach dem Trocknen der Zubereitung auf der Kopfhaut haftende, flexible Filme bilden, die dazu geeignet sind, die eingesetzten Wirkstoffe gezielt über einen bestimmten Zeitraum abzugeben. Darüber hinaus wird durch die erfindungsgemäßen Zubereitungen die unerwünschte Ausfällung des Wirkstoffes an der Applikationsstelle verhindert.

Die Erfindung betrifft daher eine pharmazeutische Zubereitung, enthaltend mindestens einen physiologisch verträglichen Filmbildner, mindestens ein physiologisch verträgliches Lösemittel, mindestens einen Weichmacher und eine Verbindung der Formel I 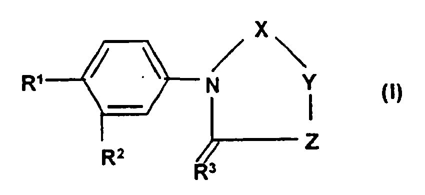
und /oder eine stereoisomere Form der Verbindung der Formel I und/oder ein physiologisch verträgliches Salz der Verbindung der Formel I, wobei
- R¹ für: 1) -CN,
2) -NO₂,
3) Halogen oder
4) -(C₁-C₄)-Alkyl-C(O)-OH steht,
- R² für: 1) -CF₃,
2) Halogen oder
3) -CN steht,
- R³ für: 1) =O,
2) =S oder
3) =NH steht,
- X für: 1) den Rest der Teilformel II 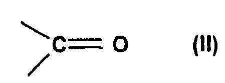
2) oder den Rest der Teilformel III steht

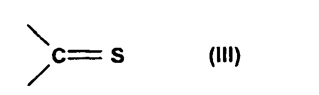

oder X und Y zusammen die Teilformel IV bilden 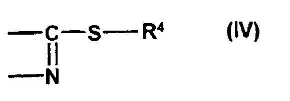
wobei R⁴ für 1) Wasserstoffatom,
2) (C₁-C₆)-Alkyl-,
3) (C₂-C₆)-Alkenyl- oder
4) (C₁-C₆)-Alkyl- steht, worin Alkyl ein- bis dreifach substituiert ist durch
   4.1 -OH,
   4.2 Halogen,
   4.3 -O-(C₁-C₄)-Alkyl,
   4.4 -CN oder
   4.5 -SH,

- Y für: 1) den Rest der Teilformel V steht , 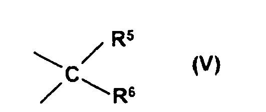
wobei R⁵ Wasserstoffatom oder (C₁-C₄)-Alkyl bedeutet, worin Alkyl unsubstituiert oder ein- bis vierfach durch Halogen substituiert ist und R⁶ für (C₁-C₄)-Alkyl steht, worin Alkyl unsubstituiert oder ein- bis dreifach unabhängig voneinander substituiert ist durch
a) Halogen,
b) Phenyl-(CH₂)ₘ-, worin Phenyl unsubstituiert oder ein- bis dreifach unabhängig voneinander substituiert ist durch -COOH, -CN oder -CF₃ und m die ganze Zahl Null,1, 2, 3, 4, 5 oder 6 bedeutet,
c) -COOH,
d) -CN oder
e) -CF₃, oder

2) den Rest der Teilformel VI steht, 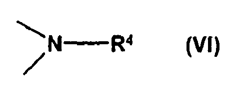
   wobei R⁴ die obengenannte Bedeutung hat, und
- Z für: 1) -O- oder
2) den Rest der Teilformel VII, 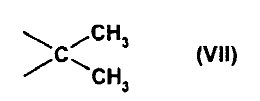
   steht.

Bevorzugt ist eine pharmazeutische Zubereitung, enthaltend eine Verbindung der Formel I, wobei
- R¹ für: 1) -CN,
2) -NO₂ oder
3) Halogen steht,
- R² für 1): -CF₃ oder
2) Halogen steht,
- R³ für 1): =O oder
2) =S steht,
- X für: den Rest der Teilformel II oder III steht oder
- X und Y: zusammen die Teilformel IV bilden,
worin R⁴ die obengenannte Bedeutung hat,
- Y für: den Rest der Teilformel VI steht,
worin R⁴ die obengenannte Bedeutung hat, und
- Z für: den Rest der Teilformel VII steht.

Insbesondere bevorzugt ist eine pharmazeutische Zubereitung, enthaltend eine Verbindung der Formel I, wobei
- R¹ für: -CN steht,
- R² für: -CF₃ steht,
- R³ für: =0 steht,
- X für: den Rest der Teilformel II steht,
- Y für: den Rest der Teilformel VI steht und worin R⁴ Wasserstoffatom bedeutet, und
- Z für: -O- oder den Rest der Teilformel VII steht.

Ganz besonders bevorzugt sind Verbindungen der Formel I genannt wie 4-[3-(4-Hydroxybutyl)-4,4-dimethyl-2,5-dioxo-1-imidazolidinyl]-2-(trifluormethyl)-benzonitril oder 4-(5-methyl-2,4-dioxo-5-trifluormethyl)-oxazolidin-3-yl)-2-(trifluormethyl)-benzonitril.

Unter dem Begriff "Halogen" wird Fluor, Chlor, Brom oder Jod verstanden. Unter dem Begriff "Alkyl" oder "Alkenyl" werden Kohlenwasserstoffreste verstanden deren Kohlenstoffketten geradkettig oder verzweigt sind. Ferner können die Alkenylreste auch mehrere Doppelbindungen enthalten. Unter dem Begriff "physiologisch verträgliches Lösemittel" werden beispielsweise Wasser, (C₁-C₆)-Alkohole wie Methanol, Ethanol, Propanol, Isopropanol, Butanol, Pentanol oder Hexanol verstanden. Es können aber auch Mischungen der Lösemittel eingesetzt werden.

Weichmacher sind Stoffe, die spröden Massen, z.B. filmbildenden Substanzen, Geschmeidigkeit und Flexibilität verleihen. Durch Art und Menge des zugesetzten Weichmachers kann darüber hinaus auch das Freisetzungsprofil von Substanzen aus Filmen gesteuert werden. Als Weichmacher kommen verschiedene Substanzklassen in Betracht, insbesondere ethoxylierte Verbindungen, Panthenol und Ester der Adipin- oder Sebacinsäure.

Filmbildner sind Stoffe unterschiedlicher Zusammensetzung, die dadurch charakterisiert sind, daß sie in Wasser oder anderen geeigneten Lösemitteln gelöst nach dem Verdunsten des Wassers oder des Lösemittels Filme auf der Haut bilden, die unter anderem dazu geeignet sind, inkorporierte Wirkstoffe über einen bestimmten Zeitraum gezielt freizusetzen.

Im Gegensatz zu Verdickungsmittel, welche flüssigen Zubereitungen zugesetzt werden, um eine bestimmte Viskosität einzustellen, beeinflussen Filmbildner die Viskosität einer Flüssigkeit nur in geringen Maße. Nachteil von Verdickungsmitteln ist die schlechte Verteilbarkeit der Applikationsform.

Die erfindungsgemäßen Zubereitungen zeichnen sich in erster Linie durch eine gleichmäßige über einen bestimmten Zeitraum ablaufende Freigabe der Verbindung der Formel I aus dem - sich nach dem Auftragen der Zubereitung bildenden - elastischen, auf der Haut gut haftenden, Film aus. Dadurch wird sichergestellt, daß therapeutisch wirksame Antiandrogen-Konzentrationen an dem Zielorgan - der Haarwurzel - über einen längeren Zeitraum erreicht werden, ohne daß kurzfristig hohe Blutspiegelkonzentrationen auftreten, die naturgemäß zu einer systemischen Belastung des Patienten führen.

Vorzugsweise handelt es sich bei den pharmazeutischen Zubereitungen um flüssige Zubereitungen wie Haarwässer oder Haartonika, die als Hauptbestandteile Wasser, aber auch wäßrigen (C₁-C₆)-Alkohol, wie beispielsweise Ethanol, Propanol oder Isopropanol enthalten können, ferner um Lotionen oder um halbfeste Zubereitungen wie Emulsionen, Cremes, Gele oder Salben. Gegebenenfalls können die Zubereitungen auch in Aerosolform vorliegen.

Als Filmbildner eignen sich z.B. natürliche Stoffe wie Alginsäure / Alginate, Kollagen / Kollagenderivate, hydrolisierte Weizenproteine, Carrageenan, Cellulose / Cellulosederivate, Chitosan / Chitosanderivate, Keratinhydrolysate, Proteinhydrolysate, Gelatine, Guar / Guarderivate, hydrolisiertes Elastin, hydrolisierte Milchproteine, hydrolisierte Seidenproteine, hydrolisiertes Sojaprotein, hydrolisierte Haferproteine, Copolymer aus Hydroxyethylcellulose und Dimethyldiallylammoniumchlorid, Hyaluronsäure / Hyaluronate, Tragacanth, Xanthan, sowie synthetische Stoffe wie Acrylat / Acrylamid Copolymere, Acrylat Copolymere, Acrylat / Octylacrylamid Copolymere, Acrylsäureester Copolymere, Methacrylsäure Copolymer, Adipinsäure / Dimethylaminohydroxypropyldiäthylentriamin Copolymere, Methacrylsäure / Methacrylsäureester Copolymere neutralisiert mit 2-Amino-2-methylpropanol, Polyacrylsäure quervernetzt mit Pentaerythritol- oder Zuckerallyläthem, Polysiloxan / Polyalkylpolyäther Copolymere, Polysiloxane, Äthylen / Acrylsäureester Copolymere, Äthylen / Vinylacetat Copolymere, Methacryloyläthylbetain / Methacrylsäure Copolymere, Octylacrylamid / Acrylsäureester / Butylaminoäthylmethacrylsäure Copolymere, Polyvinylpyrrolidondimethylaminoäthylmethacrylsäureester quaternisiert, Polyvinylpyrrolidon / Imidazoliniummethochlorid Copolymere, Natriumacrylat / Dimethyldiallylammoniumchlorid Copolymere, Dimethyldiallylammoniumchlorid / Natriumacrylat / Acrylamid Terpolymer, Poly(dimethylsiloxancopolyolphosphopanthenoat), Poly(methylvinyläthermaleinanhydrid), Poly(methylvinyläthermaleinsäuremonoalkylester), Poly(vinylpyrrolidon), Terpolymere auf der Basis von Pyrrolidon und Acrylsäureverbindungen, Poly(vinylpyrrolidondimethylaminoäthylmethacrylsäure), Polyvinylpyrrolidon / Eicosen Copolymer, Polyvinylpyrrolidon / Methacrylsäureester / Methacrylsäure Terpolymer, Polyvinylpyrrolidon / Hexadecen Copolymer, Polyvinylpyrrolidon / Polycarbamyl Polyglycolester, Polyvinylpyrrolidon / Vinylacetat Copolymer, Vinylimidazoliummethochlorid / Vinylpyrrolidon Copolymer, Acrylsäure / Acrylsäureester Copolymere und Terpolymer von Vinylpyrrolidon, Vinylacetat und Vinylpropionat.

Als Zusatzstoffe können die erfindungsgemäßen Zubereitungen auch mindestens eine durchblutungsfördernde Verbindung enthalten wie Dihydralazin, Diisopropylamin, Diazoxid oder Calciumantagonisten wie Nifedipin, Nicardipin, Verapamil, Diltiazem, Nisoldipin, Nitrendipin, Nivaldipin, Isradipin, Felodipin, Nimodipin, Gallopamil, Fendilin, Flunarizin, Amlodipin, Diperdipin, Fluspirilen, Primozid, Fantofaron, Nicergolin oder Cyclandelat, 6-Amino-4-piperidino-1,2-dihydro-1-hydroxy-2-iminopyrimidin (Minoxidil), Angiotensin-Converting-Enzym-Hemmer wie Quinapril, Lisinopril, Benzazepril, Captopril, Ramipril, Fosinopril, Cifazapril oder Trandolapril, Methylxanthinverbindungen wie Pentoxifyllin, Propentofyllin, Torbafyllin oder deren Mischung.
Geeignete Zusatzstoffe sind auch mindestens ein Natriumkanalöffner wie 1-Cyan-2-(1,1-dimethyl-propyl)-3-(3-pyridyl)-guanidin oder 5-alpha-Reduktasehemmer wie N-tertiär-Butyl-3-oxo-4aza-5α-androst-1-en-17β-carboxamid. Weitere geeignete Zusatzstoffe sind auch mindestens eine haarwachstumsfördernde Verbindung wie ein inneres Salz von 2,4-Diamino-6-alkoxy-3-sulfoxypyrimidinhydroxid mit 1 bis 6 Kohlenstoffatomen im Alkoxyrest wie beschrieben in EP 0 427 625; z.B. das innere Salz von 2,4-Diamino-6-butoxy-3- sulfoxypyrimidinhydroxid, oder Pyridin-1-oxid-derivate wie beschrieben in WO 92 21317; z.B. 2,6-Diamino-4-piperidinopyridin, oder 2,6-Diamino-1,3,5-triazin-derivate wie beschrieben in WO 91 19701; z.B. 2,6-Diamino-4-butoxy-1,3,5-triazin-1-oxid. Ferner sind auch Mischungen der genannten Zusatzstoffe geeignet.
Als weitere Zusatzstoffe können die erfindungsgemäßen Zubereitungen die in der Kosmetik üblichen haar- und kopfhautpflegenden Substanzen und medizinische Wirkstoffe enthalten wie beispielsweise Antischuppenmittel, antiseborrhoeisch wirksame Präparate, Stoffe mit keratolytischer und keratoplastischer Wirkung wie Salicylsäure, Allantoin, Schwefelpräparate, Harnstoff, Ceramide, Antimikrobica, Vitamine, Pflanzen- oder Organextrakte, Hormone, Corticoide, Hyperämica, wie Nikotinsäure und deren Derivate, organische Säuren wie Zitronensäure, Orotsäure, Liponsäure, Aminosäuren, polyoxäthylierte Fettalkohole, Fettsäuren, Sorbitanfettsäureester, Alkylphosphate und Öle, z.B. Fettsäureester, sowie ferner Konservierungsmittel, Farbstoffe und Parfümöle. Wesentlich ist, daß die Zusatzstoffe mit antiandrogen Substanzen kompatibel sind und deren Haarwuchswirkung nicht inhibieren.

Mit den erfindungsgemäßen Zubereitungen läßt sich die Behandlung der androgenetischen Alopezie sicher und wirksam durchführen. Im Hinblick auf die bisherigen schlechten Therapieerfahrungen ist dies ein überaus wichtiger Befund.

Die erfindungsgemäßen Zubereitungen sind auch zur Behandlung des Hirsutismus, das heißt zur Vermeidung von unerwünschter Behaarung und zur Behandlung der Seborrhö und Akne geeignet.

In den erfindungsgemäßen Zubereitungen ist der Wirkstoff im allgemeinen in einer Menge von 0,01 Gewichtsprozent bis 10 Gewichtsprozent, vorzugsweise 0,1 bis 5 Gewichtsprozent enthalten.

In flüssigen Zubereitungen beträgt die Menge an Lösemitteln von 85 Gewichtsprozent bis 97,5 Gewichtsprozent und die Weichmachermenge von 0,05 Gewichtsprozent bis 2,5 Gewichtsprozent. Halbfeste Zubereitungen enthalten an Lösemitteln 50 Gewichtsprozent bis 75 Gewichtsprozent und die Weichmachermenge beträgt von 0,05 Gewichtsprozent bis 2,5 Gewichtsprozent.

Die Erfindung betrifft ferner den Einsatz der erfindungsgemäßen Zubereitungen in der Kosmetik.

Im allgemeinen erfolgt die Herstellung der erfindungsgemäßen Zubereitungen in an sich bekannter Weise durch Auflösen der antiandrogen wirksamen Substanzen in dem jeweils in Frage kommenden Vehikel.

Die erfindungsgemäße Zubereitung weist beispielsweise folgende Zusammensetzung auf:

### Beispiel 1

| | |
|---|---|
| 4-[3-(4-Hydroxybutyl)-4,4-dimethyl-2,5-dioxo-1-imidazolidinyl]-2-(trifluoromethyl)-benzonitril | 5,0 % |
| Vinylimidazoliummethochlorid / Vinylpyrrolidon Copolymer | 2,5 % |
| (Luviquart® FC 550) | |
| Polyoxäthyliertes hydriertes Rizinusöl (Cremophor® RH 410) | 2,5 % |
| Ethanol 96 % | 63,0 % |
| | |
| Entmineralisiertes Wasser | 27,0 % |

Die prozentualen Mengenangaben sind auf das Gewicht bezogen.

Die Zubereitung wird durch Lösen der verschiedenen Komponenten in Wasser hergestellt.

### Beispiel 2

| | |
|---|---|
| 4-[3-(4-Hydroxybutyl)-4,4-dimethyl-2,5-dioxo-1-imidazolidinyl]-2-(trifluoromethyl)-benzonitril | 1,0 % |
| Ethoxyliertes Cholesterin | 1,0 % |
| (Solulan® C-24) | |
| Polyvinylpyrrolidon K 30 | 2,0 % |
| Partiell hydrolisiertes Kollagen | 1,5 % |
| (Lanasan CL®) | |
| Ethylalkohol 96 % | 20,0 % |
| Konservierungsmittel | |
| Entmineralisiertes Wasser | 74,5 % |

### Beispiel 3

| | |
|---|---|
| 4-[3-(4-Hydroxybutyl)-4,4-dimethyl-2,5-dioxo-1-imidazolidinyl]-2-(trifluoromethyl)-benzonitril | 0,5 % |
| Ethylalkohol | 25,0 % |
| Methylvinyläther / Maleinsäurebutylester Copolymer | 1,5% |
| (Gantrez® ES-425) | |
| Tris(hydroxymethyl)aminomethan | 0,03 % |
| Panthenol | 0,5 % |
| Entmineralisiertes Wasser | 72,47 % |

### Beispiel 4

| | |
|---|---|
| 4-[3-(4-Hydroxybutyl)-4,4-dimethyl-2,5-dioxo-1-imidazolidinyl]-2-(trifluoromethyl)-benzonitril | 2,0 % |
| Vinylimidazoliummethochlorid / Vinylpyrrolidon Copolymer | 2,0 % |
| (Luviquart® FC 550) | |
| Polyoxäthyliertes hydriertes Rizinusöl | 2,0 % |
| (Cremophor® RH 410) | |
| Ethanol 96 % | 40,0 % |
| Entmineralisiertes Wasser | 54,0 % |

### Beispiel 5

| | |
|---|---|
| 4-(5-methyl-2,4-dioxo-5-trifluoromethyl)-oxazolidin-3-yl)-2-trifluoromethylbenzonitril | 2,0 % |
| Vinylimidazoliummethochlorid / Vinylpyrrolidon Copolymer | 2,0 % |
| (Luviquart® FC 550) | |
| Polyoxäthyliertes hydriertes Rizinusöl | 2,0 % |
| (Cremophor® RH 410) | |
| Ethanol 96 % | 40,0 % |
| Entmineralisiertes Wasser | 54,0 % |

Die verzögerte Wirkstofffreisetzung aus den erfindungsgemäßen Zubereitungen wird in Permeationstests an behaarter und unbehaarter menschlicher Haut nachgewiesen. Die angewandte Meßmethode erlaubt es, die Freigabe eines Wirkstoffes aus einer bestimmten Zubereitung und die sich daran anschließende Permeation durch menschliche Haut zu prüfen.

Als Kontrollbeispiel wird

| | |
|---|---|
| 4-[3-(4-Hydroxybutyl)-4,4-dimethyl-2,5-dioxo-1-imidazolidinyl]-2-(trifluoromethyl)-benzonitril | 5,0 % |
| in Ethanol 96 % | 66,5 % |
| und entmineralisiertem Wasser | 28,5 % |

gelöst.

### Permeationstest an behaarter und unbehaarter Haut

Die Messung der Wirkstoffpermeation wird mittels zeitaufgelöster ATR Technik (Time resolved infrared attenuated total reflection - siehe Th. M. Bayerl et al.; J. Invest. Dermatol. 105:291-295, 1995) durchgeführt:

Auf die Oberseite von auf dem Meßkristall liegender behaarter und unbehaarter Humanhaut werden auf einer definierten Fläche 100 µl der Prüfzubereitung (Kontrollbeispiel) aufgetragen. Die Permeation des Wirkstoffes kann anhand der für 4-[3-(4-Hydroxybutyl)-4,4-dimethyl-2,5-dioxo-1-imidazolidinyl]-2-(trifluoromethyl)-benzonitril charakteristischen IR Band bei 1323 cm⁻¹ beobachtet werden.

Dabei zeigte sich, daß sowohl durch die behaarte als auch durch die unbehaarte Haut in 24 Stunden etwa 90 % der aufgetragenen Wirkstoffmenge permeieren.

Unterschiede zwischen beiden Hautstücken ergaben sich jedoch bei der Permeationsgeschwindigkeit. Während sich die permeierte Wirkstoffmenge bei Verwendung von behaarter Haut bereits nach etwa 7 Stunden asymtotisch dem Endwert nähert, permeiert die Substanz durch unbehaarte Haut nahezu gleichmäßig über 24 Stunden.
Nach dem Auftragen einer erfindungsgemäßen Zubereitung, z.B. gemäß Beispiel 1, auf Haarfollikel-haltige Haut - wie sie bei der androgenetischen Alopezie vorliegt - konnte ebenfalls eine gleichmäßige Permeation des Wirkstoffes über 24 Stunden wie nach dem Auftragen der Kontrollzubereitung auf unbehaarter Haut erzielt werden.

Ferner kam es bei der Anwendung der erfindungsgemäßen Zubereitung im Gegensatz zur Kontrollzubereitung nach dem Abdunsten des Lösemittels nicht zu einer Ausfällung des Wirkstoffes an der Applikationsstelle.

## Patentansprüche

1. Kopfhautbehandlungsmittel, enthaltend
a) mindestens einen physiologisch verträglichen Filmbildner,
b) mindestens ein physiologisch verträgliches Lösemittel,
c) mindestens einen Weichmacher und
d) eine Verbindung der Formel I 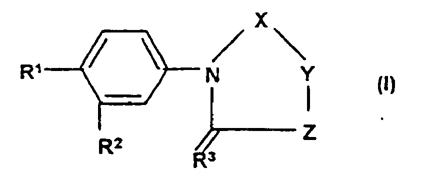
und /oder eine stereoisomere Form der Verbindung der Formel I und/oder ein physiologisch verträgliches Salz der Verbindung der Formel I, wobei
R¹ für 1) -CN,
2) -NO₂,
3) Halogen oder
4) (C₁-C₄)-Alkyl-C(O)-OH steht,
R² für 1) -CF₃,
2) Halogen oder
3) -CN steht,
R³ für 1) =O,
2) =S oder
3) =NH steht,
X für 1) den Rest der Teilformel II 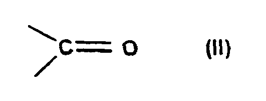
2) oder den Rest der Teilformel III steht 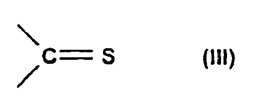
oder X und Y zusammen die Teilformel IV bilden 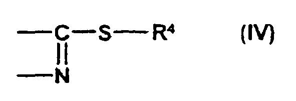
wobei R⁴ für 1) Wasserstoffatom,
2) (C₁-C₆)-Alkyl-,
3) (C₂-C₆)-Alkenyl- oder
4) (C₁-C₆)-Alkyl- steht, worin Alkyl ein- bis dreifach substituiert ist durch
4.1 -OH,
4.2 Halogen,
4.3 -O-(C₁-C₄)-Alkyl,
4.4 -CN oder
4.5 -SH,
Y für 1) den Rest der Teitformel V steht, 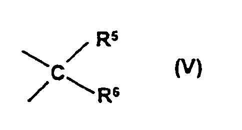
wobei R⁵ Wasserstoffatom oder (C₁-C₄)-Alkyl bedeutet, worin Alkyl unsubstituiert oder ein- bis vierfach durch Halogen substituiert ist und
R⁶ für (C₁-C₄)-Alkyl steht, worin Alkyl unsubstituiert oder ein- bis dreifach unabhängig voneinander substituiert ist durch
a) Halogen,
b) Phenyl-(CH₂)ₘ-, worin Phenyl unsubstituiert oder ein- bis dreifach unabhängig voneinander substituiert ist durch -COOH, -CN oder -CF₃ und m die ganze Zahl Null,1, 2, 3, 4, 5 oder 6 bedeutet,
c) -COOH,
d) -CN oder
e) -CF₃, oder
2) den Rest der Teilformel VI steht, 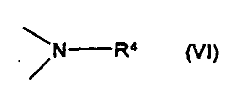
wobei R⁴ die obengenannte Bedeutung hat, und
Z für 1) -O- oder
2) den Rest der Teilformel VII,
steht.

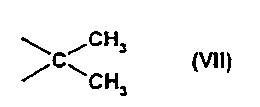

2. Kopfhautbehandlungsmittel nach Anspruch 1, enthaltend eine Verbindung der Formel I, wobei
R¹ für 1) -CN,
2) -NO₂ oder
3) Halogen steht,
R² für 1) -CF₃ oder
2) Halogen steht,
R³ für 1) =O oder
2) =S steht,
X für den Rest der Teilformel II oder III steht oder
X und Y zusammen die Teilformel IV bilden,
worin R⁴ die obengenannte Bedeutung hat,
Y für den Rest der Teilformel VI steht,
worin R⁴ die obengenannte Bedeutung hat, und
Z für den Rest der Teilformel VII steht.

3. Kopfhautbehandlungsmittel nach Anspruch 1, enthaltend eine Verbindung der Formel I, wobei
R¹ für -CN steht,
R² für -CF₃ steht,
R³ für =0 steht,
X für den Rest der Teilformel II steht,
Y für den Rest der Teilformel VI steht und worin R⁴ Wasserstoffatom bedeutet, und
Z für -O- oder den Rest der Teilformel VII steht.

4. Kopfhautbehandlungsmittel nach Anspruch 1 oder 2, enthaltend 4-[3-(4-Hydroxybutyl)-4,4-dimethyl-2,5-dioxo-1-imidazolidinyl]-2-(trifluormethyl)-benzonitril oder 4-(5-methyl-2,4-dioxo-5-trifluormethyl)-oxazolidin-3-yl)-2-(trifluormethyl)-benzonitril.

5. Kopfhautbehandlungsmittel nach einem oder mehreren der Ansprüche 1 bis 4, enthaltend als Weichmacher eine ethoxylierte Verbindung, Panthenol oder einen Ester der Adipin- oder Sebacinsäure, insbesondere polyoxyäthyliertes Ricinusöl, ethöxyliertes Cholesterin oder Panthenol.

6. Kopfhautbehandlungsmittel nach einem oder mehreren der Ansprüche 1 bis 5, enthaltend als Lösemittel Wasser, (C₁-C₆)-Alkohole wie Methanol, Ethanol, Propanol, Isopropanol, Butanol, Pentanol oder Hexanol oder Mischungen der Lösemittel.

7. Kopfhautbehandlungsmittel nach einem oder mehreren der Ansprüche 1 bis 6, enthaltend als
Filmbildner natürliche Stoffe wie Alginsäure / Alginate, Kollagen / Kollagenderivate, hydrolisierte Weizenproteine, Carrageenan, Cellulose / Cellulosederivate, Chitosan / Chitosanderivate, Keratinhydrolysate, Proteinhydrolysate, Gelatine, Guar / Guarderivate, hydrolisiertes Elastin, hydrolisierte Milchproteine, hydrolisierte Seidenproteine, hydrolisiertes Sojaprotein, hydrolisierte Haferproteine, Copolymer aus Hydroxyethylcellulose und Dimethyldiallylammoniumchlorid, Hyaluronsäure / Hyaluronate, Tragacanth, Xanthan, sowie synthetische Stoffe wie Acrylat / Acrylamide Copolymere, Acrylat Copolymere, Acrylat / Octylacrylamid Copolymere, Acrylsäureester Copolymere, Methacrylsäure Copolymer, Adipinsäure / Dimethylaminohydroxypropyldiäthylentriamin Copolymere, Methacrylsäure / Methacrylsäureester Copolymere neutralisiert mit 2-Amino-2-methylpropanol, Polyacrylsäure quervemetzt mit Pentaerythritol- oder Zuckerallyläthem, Polysiloxan / Polyalkylpolyäther Copolymere, Polysiloxane, Äthylen / Acrylsäureester Copolymere, Äthylen / Vinylacetat Copolymere, Methacryloyläthylbetain / Methacrylsäure Copolymere, Octylacrylamid / Acrylsäureester / Butylaminoäthylmethacrylsäure Copolymere, Polyvinylpyrrolidondimethylaminoäthylmethacrylsäureester quatemisiert, Polyvinylpyrrolidon / Imidazoliniummethochlorid Copolymere, Natriumacrylat / Dimethyldiallylammoniumchlorid Copolymere, Dimethyldiallylammoniumchlorid / Natriumacrylat / Acrylamid Terpolymer, Poly(dimethylsiloxancopolyolphosphopanthenoat), Poly(methylvinyläthermaleinanhydrid), Poly(methylvinyläthermaleinsäuremonoalkylester), Poly(vinylpyrrolidon), Terpolymere auf der Basis von Pyrrolidon und Acrylsäureverbindungen, Poly(vinylpyrrolidondimethylaminoäthylmethacrylsäure), Polyvinylpyrrolidon / Eicosen Copolymer, Polyvinylpyrrolidon / Methacrylsäureester / Methacrylsäure Terpolymer, Polyvinylpyrrolidon / Hexadecen Copolymer, Polyvinylpyrrolidon / Polycarbamyl Polyglycolester, Polyvinylpyrrolidon / Vinylacetat Copolymer, Vnylimidazoliummethochlorid / Vinylpyrrolidon Copolymer, Acrylsäure / Acrylsäureester Copolymere und Terpolymer von Vinylpyrrolidon, Vinylacetat und Vinylpropionat.

8. Kopfhautbehandlungsmittel nach einem oder mehreren der Ansprüche 1 bis 7, die als weiteren Zusatzstoff mindestens eine durchblutungsfördernde Verbindung wie
Dihydralazin, Diisopropylamin oder Diazoxid oder Calciumantagonisten wie Nifedipin, Nicardipin, Verapamil, Diltiazem, Nisoldipin, Nitrendipin, Nivaldipin, Isradipin, Felodipin, Nimodipin, Gallopamil, Fendilin, Flunarizin, Amlodipin, Diperdipin, Fluspirilen, Primozid, Fantofaron, Nicergolin oder Cyclandelat, 6-Amino-4-piperidino-1,2-dihydro-1-hydroxy-2-iminopyrimidin (Minoxidil), Angiotensin-Converting-Enzym-Hemmer wie Quinapril, Lisinopril, Benzazepril, Captopril, Ramipril, Fosinopril, Cifazapril oder Trandolapril, Methylxanthinverbindungen wie Pentoxifyllin, Propentofyllin oder Torbafyllin oder deren Mischung enthält oder mindestens ein Natriumkanalöffner wie 1-Cyan-2-(1,1-dimethyl-propyl)-3-(3-pyridyl)-guanidin oder 5-alpha-Reduktasehemmer wie N-tertiär-Butyl-3-oxo-4aza-5α-androst-1-en-17β-carboxamid oder mindestens eine haarwachstumsfördemde Verbindung wie innere Salze von 2,4-Diamino-6-alkoxy-3-sulfoxypyrimidinhydroxid mit-1 bis 6 Kohlenstoffatomen im Alkoxyring wie das innere Salz von 2,4-Diamino-6-butoxy-3- sulfoxypyrimidinhydroxid oder Pyridin-1-oxid-derivate wie 2,6-Diamino-4-piperidinopyridin oder 2,6-Diamino-1,3,5-triazin-derivate wie 2,6-Diamino-4-butoxy-1,3,5-triazin-1-oxid oder Mischungen davon enthält.

9. Verwendung eines Kopfhautbehandlungsmittel nach einem oder mehreren der Ansprüche 1 bis 8 zur Herstellung eines Arzneimittels zur Behandlung der androgenetischen Alopezie, des Hirsutismus, das heißt zur Vermeidung von unerwünschter Behaarung und zur Behandlung der Seborrhö und Akne.

10. Nicht-therapeutische Verwendung eines Kopfhautbehandlungsmittel nach einem oder mehreren der Ansprüche 1 bis 8 in der Kosmetik.

## Claims

1. Scalp treatment composition comprising
a) at least one physiologically tolerated film-forming agent,
b) at least one physiologically tolerated solvent,
c) at least one plasticizer and
d) a compound of the formula I 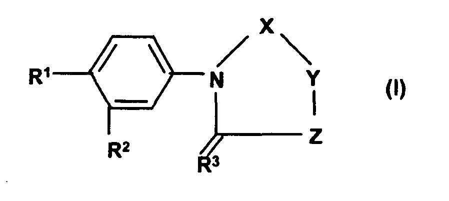
and /or a stereoisomeric form of the compound of the formula I and/or a physiologically tolerated salt of the compound of the formula I, in which
R¹ is 1) -CN,
2) -NO₂,
3) halogen or
4) (C₁-C₄)-alkyl-C(O)-OH,
R² is 1) -CF₃,
2) halogen or
3) -CN,
R³ is 1) =O,
2) =S or
3) =NH,
X is 1) the radical of the part formula II 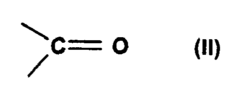
or
2) the radical of the part formula III 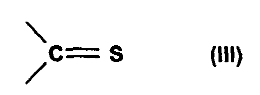
or
X and Y together form the part formula IV 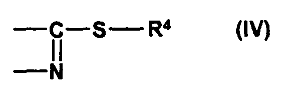
in which R⁴ is 1) hydrogen atom,
2) (C₁-C₆)-alkyl-,
3) (C₂-C₆)-alkenyl- or
4) (C₁-C₆)-alkyl-, in which alkyl is mono- to trisubstituted by
4.1 -OH,
4.2 halogen,
4.3 -O-(C₁-C₄)-alkyl,
4.4 -CN or
4.5 -SH,
Y is 1) the radical of the part formula V 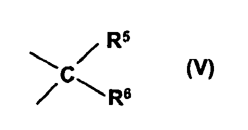
in which R⁵ is a hydrogen atom or (C₁-C₄)-alkyl, in which alkyl is unsubstituted or mono- to tetrasubstituted by halogen, and
R⁶ is (C₁-C₄)-alkyl, in which alkyl is unsubstituted or mono- to trisubstituted, independently of one another, by
a) halogen,
b) phenyl-(CH₂)ₘ-, in which phenyl is unsubstituted or mono- to trisubstituted, independently of one another, by -COOH, -CN or -CF₃ and m is the integer zero, 1, 2, 3, 4, 5 or 6,
c) -COOH,
d) -CN or
e) -CF₃, or
2) the radical of the part formula VI, 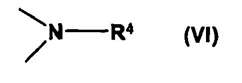
in which R⁴ has the abovementioned meaning, and
Z is 1) -O- or
2) the radical of the part formula VII

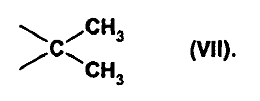

2. Scalp treatment composition according to Claim 1, comprising a compound of the formula I in which
R¹ is 1) -CN,
2) -NO₂ or
3) halogen,
R² is 1) -CF₃ or
2) halogen,
R³ is 1) =O or
2) =S,
X is the radical of the part formula II or III or
X and Y together form the part formula IV,
in which R⁴ has the abovementioned meaning,
Y is the radical of the part formula VI,
in which R⁴ has the abovementioned meaning, and
Z is the radical of the part formula VII.

3. Scalp treatment composition according to Claim 1, comprising a compound of the formula I in which
R¹ is -CN,
R² is -CF₃,
R³ is =0,
X is the radical of the part formula II,
Y is the radical of the part formula VI and in which R⁴ is a hydrogen atom, and
Z is -O- or the radical of the part formula VII.

4. Scalp treatment composition according to Claim 1 or 2, comprising 4-[3-(4-hydroxybutyl)-4,4-dimethyl-2,5-dioxo-1-imidazolidinyl]-2-(trifluoromethyl)benzonitrile or 4-(5-methyl-2,4-dioxo-5-trifluoromethyl)-oxazolidin-3-yl)-2-(trifluoromethyl)-benzonitrile.

5. Scalp treatment composition according to one or more of Claims 1 to 4, comprising an ethoxylated compound, panthenol or an ester of adipic acid or sebacic acid, in particular polyoxyethylated castor oil, ethoxylated cholesterol or panthenol, as the plasticizer.

6. Scalp treatment composition according to one or more of Claims 1 to 5, comprising water or (C₁-C₆)-alcohols, such as methanol, ethanol, propanol, isopropanol, butanol, pentanol or hexanol, or mixtures of the solvents, as the solvent.

7. Scalp treatment composition according to one or more of Claims 1 to 6, comprising naturally occurring substances, such as alginic acid / alginates, collagen / collagen derivatives, hydrolysed wheat proteins, carrageenan, cellulose / cellulose derivatives, chitosan / chitosan derivatives, keratin hydrolysates, protein hydrolysates, gelatin, guar gum/ guar gum derivatives, hydrolysed elastin, hydrolysed milk proteins, hydrolysed silk proteins, hydrolysed soya protein, hydrolysed oat proteins, copolymer of hydroxyethylcellulose and dimethyldiallylammonium chloride, hyaluronic acid / hyaluronates, tragacanth and xanthan, and synthetic substances, such as acrylate / acrylamide copolymers, acrylate copolymers, acrylate / octylacrylamide copolymers, acrylic acid ester copolymers, methacrylic acid copolymers, adipic acid / dimethylaminohydroxypropyldiethylenetriamine copolymers, methacrylic acid / methacrylic acid ester copolymers neutralized with 2-amino-2-methylpropanol, polyacrylic acid crosslinked with pentaerythritol ethers or sugar allyl ethers, polysiloxane / polyalkyl polyether copolymers, polysiloxanes, ethylene / acrylic acid ester copolymers, ethylene / vinyl acetate copolymers, methacryloylethylbetaine / methacrylic acid copolymers, octylacrylamide / acrylic acid ester / butylaminoethylmethacrylic acid copolymers, quatemized polyvinylpyrrolidone-dimethylaminoethylmethacrylic acid esters, polyvinylpyrrolidone / imidazolinium methochloride copolymers, sodium acrylate / dimethyldiallylammonium chloride copolymers, dimethyldiallylammonium chloride / sodium acrylate / acrylamide terpolymer, poly(dimethylsiloxane-copolyol-phosphopanthenoate), poly(methyl vinyl ether-maleic anhydride), poly(methyl vinyl ether-maleic acid monoalkyl ester), poly(vinylpyrrolidone), terpolymers based on pyrrolidone and acrylic acid compounds, poly(vinylpyrrolidone-dimethylaminoethylmethacrylic acid), polyvinylpyrrolidone / eicosene copolymer, polyvinylpyrrolidone / methacrylic acid ester / methacrylic acid terpolymer, polyvinylpyrrolidone / hexadecene copolymer, polyvinylpyrrolidone / polycarbamyl polyglycol ester, polyvinylpyrrolidone / vinyl acetate copolymer, vinylimidazolium methochloride / vinylpyrrolidone copolymer, acrylic acid / acrylic acid ester copolymers and terpolymer of vinylpyrrolidone, vinyl acetate and vinyl propionate, as the film-forming agent.

8. Scalp treatment composition according to one or more of Claims 1 to 7, which comprises as a further additive at least one circulation-promoting compound, such as dihydralazine, diisopropylamine or diazoxide, or calcium antagonists, such as nifedipine, nicardipine, verapamil, diltiazem, nisoldipine, nitrendipine, nivaldipine, isradipine, felodipine, nimodipine, gallopamil, fendiline, flunarizine, amlodipine, diperdipine, fluspirilene, primozide, fantofarone, nicergoline or cyclandelate, 6-amino-4-piperidino-1,2-dihydro-1 -hydroxy-2-iminopyrimidine (minoxidil), angiotensin converting enzyme inhibitors, such as quinapril, lisinopril, benzazepril, captopril, ramipril, fosinopril, cifazapril or trandolapril, methylxanthine compounds, such as pentoxifyllin, propentofyllin or torbafyllin, or a mixture thereof, or comprises at least one sodium channel opener, such as 1-cyano-2-(1,1-dimethyl-propyl)-3-(3-pyridyl)guanidine, or 5-alpha-reductase inhibitors, such as N-tert-butyl-3-oxo-4aza-5α-androst-1-ene-17β-carboxamide, or at least one hair growth-promoting compound, such as inner salts of 2,4-diamino-6-alkoxy-3-sulphoxypyrimidine hydroxide having 1 to 6 carbon atoms in the alkoxy radical, such as the inner salt of 2,4-diamino-6-butoxy-3-sulphoxypyrimidine hydroxide, or pyridine 1-oxide derivatives, such as 2,6-diamino-4-piperidinopyridine, or 2,6-diamino-1,3,5-triazine derivatives, such as 2,6-diamino-4-butoxy-1,3,5-triazine 1-oxide or mixtures thereof.

9. Use of a scalp treatment composition according to one or more of Claims 1 to 8 for the preparation of a medicament for treatment of androgenic alopecia or hirsutism, i.e. for avoiding undesirable hair growth, and for treatment of seborrhea and acne.

10. Non-therapeutic use of a scalp treatment composition according to one or more of Claims 1 to 8 in cosmetics.

## Revendications

1. Composition de traitement du cuir chevelu, contenant
a) au moins un agent filmogène physiologiquement acceptable,
b) au moins un solvant physiologiquement acceptable,
c) au moins un assouplissant et
d) un composé de formule I 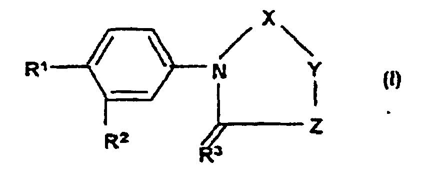
et/ou une forme stéréoisomère du composé de formule I et/ou un sel physiologiquement acceptable du composé de formule I, formule dans laquelle
R¹ représente
1) -CN,
2) -NO₂,
3) un atome d'halogène ou
4) un groupe alkyl (C₁-C₄) -C (O) -OH,
R² représente
1) -CF₃,
2) un atome d'halogène ou
3) -CN,
R³ représente
1) =O,
2) =S ou
3) =NH,
X représente
1) le radical de formule partielle II 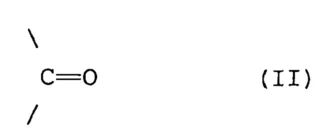
2) ou le radical de formule partielle III 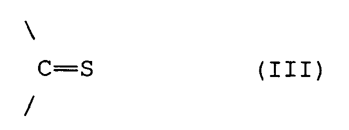
ou X et Y forment ensemble la formule partielle IV 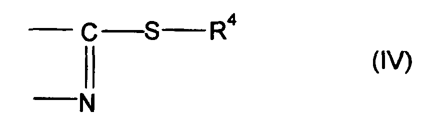
R⁴ représentant
1) un atome d'hydrogène,
2) un groupe alkyle en C₁-C₆,
3) un groupe alcényle en C₂-C₆ ou
4) un groupe alkyle en C₁-C₆, le groupe alkyle étant une à trois fois substitué par
4.1 -OH,
4.2 halogène,
4.3 -O-alkyle(C₁-C₄),
4.4 -CN ou
4.5 -SH,
Y représentant
1) le radical de formule partielle V 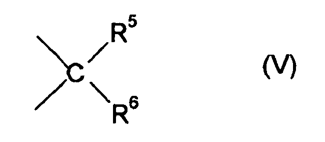
R⁵ représentant un atome d'hydrogène ou un groupe alkyle en C₁-C₄, le groupe alkyle étant non substitué ou une à quatre fois substitué par halogène, et
R6 représentant un groupe alkyle en C₁-C₄, le groupe alkyle étant non substitué ou une à trois fois substitué, indépendamment les uns des autres, par
a) halogène,
b) phényl(CH₂)ₘ-, le groupe phényle étant non substitué ou une à trois fois substitué, indépendamment les uns des autres, par -COOH, -CN ou -CF₃, et m représentant le nombre entier zéro, 1, 2, 3, 4, 5 ou 6,
c) -COOH,
d) -CN ou
e) -CF₃, ou
2) le radical de formule partielle VI 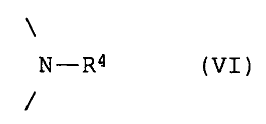
R⁴ ayant la signification donnée plus haut et
Z représente
1) -O- ou
2) le radical de formule partielle VII

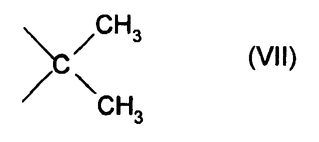

2. Composition de traitement du cuir chevelu selon la revendication 1, contenant un composé de formule I
dans lequel
R¹ représente
1) -CN,
2) -NO₂ ou
3) un atome d'halogène,
R² représente
1) -CF₃ ou
2) un atome d'halogène,
R³ représente
1) =O ou
2) =S,
X représente le radical de formule partielle II ou III ou
X et Y forment ensemble la formule partielle IV,
dans laquelle R⁴ a la signification donnée plus haut,
Y représente le radical de formule partielle VI,
dans laquelle R⁴ a la signification donnée plus haut, et
Z représente le radical de formule partielle VII.

3. Composition de traitement du cuir chevelu selon la revendication 1, contenant un composé de formule I dans lequel
R¹ représente -CN,
R² représente -CF₃,
R³ représente =O,
X représente le radical de formule partielle II,
Y représente le radical de formule partielle VI et dans lequel R⁴ représente un atome d'hydrogène, et
Z représente -O- ou le radical de formule partielle VII.

4. Composition de traitement du cuir chevelu selon la revendication 1 ou 2, contenant du 4-[3-(4-hydroxybutyl)-4,4-diméthyl-2,5-dioxo-1-imidazolidinyl]-2-(trifluorométhyl)benzonitrile ou du 4-(5-méthyl-2,4-dioxo-5-trifluorométhyl)oxazolidin-3-yl)-2-(trifluorométhyl)benzonitrile.

5. Composition de traitement du cuir chevelu selon une ou plusieurs des revendications 1 à 4, contenant en tant qu'assouplissant un composé éthoxylé, du panthénol ou un ester de l'acide adipique ou sébacique, en particulier de l'huile de ricin polyoxyéthylée, du cholestérol éthoxylé ou du panthénol.

6. Composition de traitement du cuir chevelu selon une ou plusieurs de revendications 1 à 5, contenant en tant que solvant de l'eau, des alcools en C₁-C₆ tels que le méthanol, l'éthanol, le propanol, l'isopropanol, le butanol, le pentanol ou l'hexanol, ou des mélanges des solvants.

7. Composition de traitement du cuir chevelu selon une ou plusieurs des revendications 1 à 6, contenant en tant qu'agent filmogène des substances naturelles telles que
l'acide alginique/des alginates, le collagène/des dérivés de collagène, des protéines de blé hydrolysées, le carraghénane, la cellulose/des dérivés de cellulose, le chitosane/des dérivés de chitosane, des hydrolysats de kératine, des hydrolysats de protéines, des gélatines, du guar/des dérivés de guar, de l'élastine hydrolysée, des protéines de lait hydrolysées, des protéines de soie hydrolysées, la protéine de soja hydrolysée, des protéines d'avoine hydrolysées, un copolymère d'hydroxyéthylcellulose et chlorure de diméthyldiallylammonium, l'acide hyaluronique/des hyaluronates, la gomme adragante, la gomme xanthane, ainsi que des substances synthétiques telles que des copolymères acrylate/acrylamide, des copolymères d'acrylate, des copolymères acrylate/octylacrylamide, des copolymères d'ester d'acide acrylique, un copolymère d'acide méthacrylique, des copolymères acide adipique/diméthylaminohydroxypropyldiéthylènediamine, des copolymères acide méthacrylique/ester d'acide méthacrylique neutralisés avec du 2-amino-2-méthylpropanol, un poly(acide acrylique) réticulé avec des éthers allyliques de glucides ou de pentaérythritol, des copolymères polysiloxane/polyalkylpolyéther, des polysiloxanes, des copolymères éthylène/ester d'acide acrylique, des copolymères éthylène/acétate de vinyle, des copolymères méthacryloyléthylbétaïne/acide méthacrylique, des copolymères octylacrylamide/ester d'acide acrylique/acide butylaminoéthylméthacrylique, des polyvinylpyrrolidone-méthacrylates de diméthylaminoéthyle rendus quaternaires, des copolymères polyvinylpyrrolidone/méthochlorure d'imidazolinium, des copolymères acrylate de sodium/chlorure de diméthyldiallylammonium, un terpolymère chlorure de diméthyldiallylammonium/acrylate de sodium/acrylamide, le poly(diméthylsiloxane-copolyolphosphopanthénoate), le poly(éther méthylvinyliqueanhydride maléique), le poly(éther méthylvinyliquemaléate de monoalkyle), la polyvinylpyrrolidone, des terpolymères à base de pyrrolidone et de dérivés d'acide acrylique, le poly(vinylpyrrolidoneméthacrylate de diméthylaminoéthyle), un copolymère polyvinylpyrrolidone/eicosène, un terpolymère polyvinylpyrrolidone/ester d'acide méthacrylique/acide méthacrylique, un copolymère polyvinylpyrrolidone/hexadécène, un copolymère polyvinylpyrrolidone/polycarbamyl-polyglycolester, un copolymère polyvinylpyrrolidone/acétate de vinyle, un copolymère méthochlorure de vinylimidazolium/vinylpyrrolidone, des copolymères acide acrylique/ester d'acide acrylique et un terpolymère de vinylpyrrolidone, acétate de vinyle et propionate de vinyle.

8. Composition de traitement du cuir chevelu selon une ou plusieurs des revendications 1 à 7, qui contient comme autre additif au moins un composé favorisant l'irrigation sanguine, tel que
la dihydralazine, la diisopropylamine ou le diazoxyde ou des antagonistes du calcium tels que la nifédipine, la nicardipine, la vérapamil, le diltiazem, la nisoldipine, la nitrendipine, la nivaldipine, l'isradipine, la félodipine, la nimodipine, le gallopamil, la fendiline, la flunarizine, l'amlodipine, la diperdipine, le fluspirilène, le promozide, la fantofarone, la nicergoline ou le cyclandélate, la 6-amino-4-pipéridino-1,2-dihydro-1-hydroxy-2-iminopyrimidine (minoxidil), des inhibiteurs de l'enzyme de conversion de l'angiotensine tels que le quinapril, le lisinopril, le benzazépril, le captopril, le ramipril, le fosinopril, le cifazapril ou le trandolapril, des composés méthylxanthine tels que la pentoxifylline, la propentofylline ou la torbafylline, ou des mélanges de ceux-ci, ou contient au moins un activateur du canal sodique, tel que la 1-cyano-2-(1,1-diméthylpropyl)-3-(3-pyridyl)guanidine ou des inhibiteurs de 5-α-réductase tels que le N-tert-butyl-3-oxo-4-aza-5α-androst-1-ène-17β-carboxamide, ou au moins un composé favorisant la pousse des cheveux, tels que des sels internes d'hydroxyde de 2,4-diamino-6-alcoxy-3-sulfoxypyrimidine ayant de 1 à 6 atomes de carbone dans le cycle alcoxy, comme le sel interne d'hydroxyde de 2,4-diamino-6-butoxy-3-sulfoxypyrimidine ou des dérivés de 1-oxyde de pyridine tels que la 2,6-diamino-4-pipéridinopyridine ou des dérivés de 2,6-diamino-1,3,5-triazine tels que le 1-oxyde de 2,6-diamino-4-butoxy-1,3,5-triazine ou des mélanges de ceux-ci.

9. Utilisation d'une composition de traitement du cuir chevelu selon une ou plusieurs des revendications 1 à 8, pour la fabrication d'un médicament destiné au traitement de l'alopécie androgénétique, de l'hirsutisme, c'est-à-dire pour éviter la pilosité indésirable, et au traitement de la séborrhée et de l'acné.

10. Utilisation non thérapeutique d'une composition de traitement du cuir chevelu selon une ou plusieurs des revendications 1 à 8, en cosmétique.
